# EUROPÄISCHE PATENTSCHRIFT

(11) **EP 1 515 659 B1**
(45) Veröffentlichungstag und Bekanntmachung des Hinweises auf die Patenterteilung: **05.04.2006**
(21) Anmeldenummer: 03761533.3
(22) Anmeldetag: 26.06.2003
(51) Int. Cl.: A61B 18/00

(54) **ANSCHLUSSEINRICHTUNG FÜR EIN ELEKTROCHIRURGISCHES INSTRUMENT**
CONNECTING DEVICE FOR AN ELECTROSURGICAL INSTRUMENT
DISPOSITIF DE CONNEXION DESTINE A UN INSTRUMENT ELECTROCHIRURGICAL

(30) Priorität: 27.06.2002 DE 10228791
(43) Veröffentlichungstag der Anmeldung: 23.03.2005
(73) Patentinhaber: Erbe Elektromedizin GmbH, 72072 Tübingen (DE)
(72) Erfinder: HAGG, Martin, 72827 Wannweil (DE); KÜHNER, Ralf, 70565 Stuttgart (DE); SCHNITZLER, Uwe, 72074 Tübingen (DE)
(74) Vertreter: Bohnenberger, Johannes
(86) Internationale Anmeldenummer: PCT/EP2003/006795
(87) Internationale Veröffentlichungsnummer: WO 2004/002345

(56) Entgegenhaltungen:
- DE-B- 1 159 574
- US-A- 4 781 175
- US-A- 5 061 268

## Beschreibung

Die Erfindung betrifft eine Anschlusseinrichtung für ein elektrochirurgisches Instrument, welches mindestens eine Gaszuführungsleitung und ein Stromzuführungsleitung aufweist, gemäß dem Oberbegriff des Patentanspruches 1.

Elektrochirurgische Instrumente, wie beispielsweise in der Endoskopie verwendete Argon-Plasma-Koagulationssonden (APc-sonden), weisen häufig eine Gaszuführungsleitung zum Zuführen von Gas und eine Stromzuführungsleitung zum Zuführen von HF-Strom auf. Die Sonden sind in der Regel über Anschlussleitungen mit einem die Gas- und Stromzuführung steuernden elektrischen Gerät verbunden.

Aus EP 0 447 121 A2 ist eine Sonde mit Anschlussleitungen zur Verbindung mit einem Gerät bekannt, bei der ein Zusammenführen des in voneinander getrennten Anschlussleitungen der Sonde zugeführten Gases und HF-Stromes erst an einem vorderen Spitzenbereich der Sonde stattfindet. Deshalb können insbesondere bei der Verwendung der Sonde im gastroenterologischen Bereich Sekrete und Flüssigkeiten aus menschlichen oder tierischen Körpern während einer Behandlung In die Gaszuführungs- und Stromzuführungsleitungen eindringen und diese verunreinigen. Derartige Verunreinigungen entstehen nicht nur in den innerhalb der Sonde angeordneten Leitungsabschnitten, sondern auch in den mit der Sonde verbundenen Anschlussleitungen. Dies hat zur Folge, dass nach dem Auftreten einer derartigen Verunreinigung sowohl die Sonde als auch die Anschlussleitungen ausgewechselt werden müssen.

Zwar weisen die in der EP 0 447 121 A2 gezeigten Anschlusseinrichtungen zum Anschließen der Sonde mit den Anschlussleitungen an das Gerät einen Filter auf, der als Schutz gegen derartige Verunreinigungen vorgesehen ist. Dieser Filter kann jedoch aufgrund seiner Platzierung lediglich das Gerät vor Verunreinigungen schützen.

US 4 781 175 offenbart eine APC-Sonde gemäß dem Oberbegriff von Anspruch 1.

Demzufolge liegt der vorliegenden Erfindung die Aufgabe zugrunde, eine Anschlusseinrichtung für elektrochirurgische Instrumente zur Verfügung zu stellen, die einen wirkungsvollen Schutz gegen Verunreinigungen durch Sekrete und Flüssigkeiten von menschlichen oder tierischen Körpern von mit dem Instrument verbundenen oder in dem Instrument integrierten Anschlussleitungen ermöglicht. Des weiteren ist es Aufgabe der Erfindung, eine .Verwendung für eine derartige Anschlusseinrichtung zur Verfügung zu stellen.

Diese Aufgabe wird vorrichtungsseitig durch die Merkmale des Patentanspruches 1 gelöst.

Ein wesentlicher Punkt der Erfindung ist darin zu sehen, dass eine Anschlusseinrichtung für ein elektrochirurgisches Instrument, welches eine APC Sonde ist, innerhalb eines Gehäuses eine Abzweigungseinrichtung aufweist, über welche eine in einer Gaszuführungsleitung angeordnete Stromzuführungsleitung aus der Gaszuführungsleitung zur Bildung eines Gasanschlussendstückes und eines Stromanschlussendstückes geführt wird, um einen Filter innerhalb des Gehäuses im Gasanschlussendstück anzuordnen. Das Gasanschlussendstück und das Stromanschlussendstück sind mit einem an dem Gehäuse befestigten Stecker zur Verbindung mit einer Steckbuchse in einem Gerät oder zum Gerät führenden Anschlussleitungen gekoppelt. Die Platzierung des Filters unmittelbar hinter dem chirurgischen Instrument, das als Sonde ausgebildet ist, verhindert das Eindringen von Sekreten und Flüssigkeiten in die Anschlussleitungen und/oder das Gerät, so dass sich ein Auswechseln der Anschlussleitungen nach erfolgter Operation erübrigt.

Vorteilhaft weist die Abzweigungseinrichtung eine erste Ausnehmung zur steckartigen Aufnahme der Gaszuführungsleitung mit der in dieser angeordneten Stromzu-führungsleitung und eine zweite Ausnehmung zur steckartigen Aufnahme des Filters auf. Auf diese Weise kann entweder die Abzweigungseinrichtung einschließlich des Filters oder der Filter für sich alleine nach einer vorzugsweise vorbestimmten Gebrauchzeit einfach und schnell ausgetauscht werden. Für diesen Austauschvorgang müssen das Gehäuse geöffnet, die auszutauschenden Elemente ausgewechselt und das Gehäuse wieder geschlossen werden. Somit kann der Filter auf einfache Weise ersetzt werden, bevor eine Übersättigung eines in dem Filter enthaltenen Füterelements stattfindet.

Gemäß einer bevorzugten Ausführungsform weist die Abzweigungseinrichtung einen integrierten Kabelkanal zur Aufnahme der abgezweigten Stromzuführungsleitung auf. Der Kabelkanal trägt zu einer festen Positionierung der Stromzuführungsleitung innerhalb der Abzweigungseinrichtung und damit zu einem gegenüber Beschädigungen unanfälligen Übergangsbereich der Gaszuführungsleitung bei, in welchem die Stromzuführungsleitung abgezweigt wird.

Das elektrochirurgische Instrument kann zum einmaligen Gebrauch ausgebildet sein und von der Anschlusseinrichtung mittels einer Steckverbindung schnell und einfach abgekoppelt sowie ein neues Instrument mit dieser verbunden werden.

Weitere vorteilhafte Ausführungsformen ergeben sich aus den Unteransprüchen.

Vorteile und Zweckmäßigkeiten sind der nachfolgenden Beschreibung in Verbindung mit der Zeichnung zu entnehmen. Hierbei zeigen:
- Fig. 1: in einer perspektivischen Draufsicht ausschnittweise eine Ausführungsform der erfindungsgemäßen Anschlusseinrichtung;
- Fig. 2: eine vergrößerte Darstellung eines Ausschnitts der in Figur 1 gezeigten Ausführungsform der erfindungsgemäßen Anschlusseinrichtung und
- Fig. 3: eine perspektivische Ansicht der Unterseite der in den Figuren 1 und 2 gezeigten Ausführungsformen der erfindungsgemäßen Anschlusseinrichtung.

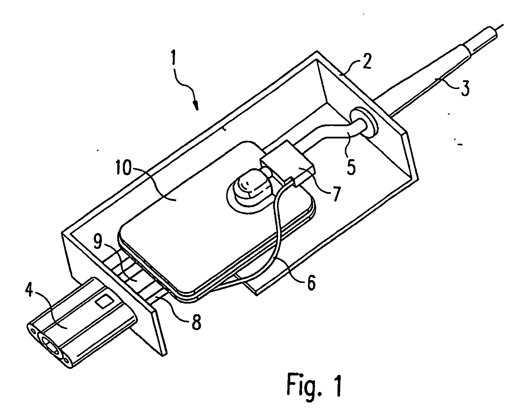

Figur 1 zeigt in einer perspektivischen Draufsicht ausschnittsweise eine Ausführungsform der erfindungsgemäßen Anschlusseinrichtung. Die Anschlusseinrichtung 1 umfasst ein hier nur teilweise dargestelltes Gehäuse, welches normalerweise in geschlossener Form vorliegt. Das Gehäuse 2 kann mittels eines Schiebe- oder Klappmechanismuses geöffnet und wieder geschlossen werden, um Zugang zu den in dem Gehäuse 2 enthaltenen Elementen, insbesondere einem Filter zu erhalten.

Eine Sonde 3 ist vorzugsweise lösbar mit dem Gehäuse an einem Gehäuseende verbunden. An einem weiteren Gehäuseende ist ein Stecker 4 zur Verbindung mit einer Steckbuchse in einem Gerät oder zum Gerät führenden Anschlussleitungen angeordnet.

Eine mit der Sonde 3 verbundene Gaszuführungsleitung 5 umschließt eine Stromzuführungsleitung 6. Beide Stromzuführungsleitungen sind mit einer Abzweigungseinrichtung 7, über welche die Stromzuführungsleitung 6 aus der Gaszuführungsleitung 5 zur Bildung eines Stromanschlussendstückes 8 und eines Gasanschlussstückes 9 geführt ist, verbunden.

Zwischen dem Gasanschlussendstück 9 und der Abzweigungseinrichtung 7 ist ein Filter 10 austauschbar angeordnet.

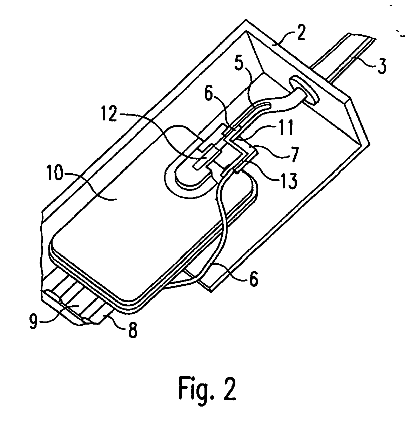

Figur 2 zeigt in einer perspektivischen Draufsicht vergrößert einen Teil der in Figur 1 gezeigten Anschlusseinrichtung. Der Figur 2 ist zu entnehmen, dass die Gaszuführungsleitung 5 und die darin angeordnete Stromzuführungsleitung 6 mit der Abzweigungseinrichtung 7 mittels einer darin angeordneten Ausnehmung 11 steckartig verbunden ist. In einer zweiten Ausnehmung 12 der Abzweigungseinrichtung 7 ist der Filter 10 eingesteckt.

Die aus der Gaszuführungsleitung 5 abgezweigte Stromzuführungsleitung 6 wird in einem innerhalb der Abzweigungseinrichtung 7 angeordneten Kabelkanal 13 derart geführt, dass sie den Filter 10 führungsfrei bis zu dem Stromanschlussendstück 8 umlaufen kann. Somit ist ein schnelles und einfaches Auswechseln des Filters 10 zwischen der Abzweigungseinrichtung 7 und eines Teils des Gasanschlussendstücks 9 möglich, ohne dass eine Unterbrechung der Stromzuführungsleitung 6 notwendig ist.

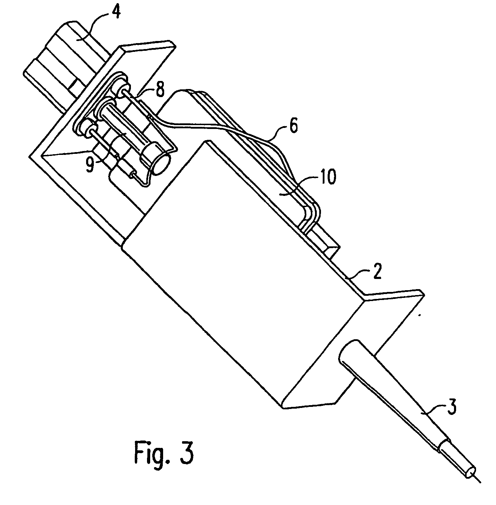

In Figur 3 wird in einer perspektivischen Darstellung die Unterseite der Ausführungsform der in den Figuren 1 und 2 gezeigten Anschlusseinrichtung gezeigt. Dieser Darstellung ist zu entnehmen, dass der Filter 10 in einen Teil des Gasanschlussendstücks 9 und die Stromzuführungsleitung 6 in das Stromanschlussendstück 8 gehäuseunterseitig münden. Somit behindern die Anschlussendstücke 8 und 9 nicht den Auswechselvorgang des Filters 10, sofern der Filter 10 gehäuseoberseitig herausgenommen bzw. wieder eingesetzt wird.

Das Gasanschlussendstück 9 und das Stromanschlussendstück 8 sind mit dem Stecker 4 zur Verbindung mit Anschlussleitungen verbunden.

An dieser Stelle sei darauf hingewiesen, dass alle oben beschriebenen Teile für sich allein gesehen und in jeder Kombination, insbesondere die in der Zeichnung dargestellten Details als Erfindung beansprucht werden. Abänderungen hiervon sind dem Fachmann geläufig.

### Bezugszeichenliste

- 1: Anschlusseinrichtung
- 2: Gehäuse
- 3: Sonde
- 4: Stecker
- 5: Gaszuführungsleitung
- 6: Stromzuführungsleitung
- 7: Abzweigungseinrichtung
- 8: Stromanschlussendstück
- 9: Gasanschlussendstück
- 10: Filter
- 11: erste Ausnehmung
- 12: zweite Ausnehmung
- 13: Kabelkanal

## Patentansprüche

1. APC-Sonde, welche mindestens eine Gaszuführungsleitung (5) aufweist, die mit einer Steckbuchse in einem Gerät oder einer zum Gerät führenden Anschlussleitung mittels eines Steckers (4) verbindbar ist, umfassend
ein Gehäuse (2) in welches die Gaszuführungsleitung (5) mit der in dieser angeordneten Stromzuführungsleitung (6) führt und mit einem Gasanschlussendstück (9) und einem Stromanschlussendstück (8),
eine Abzweigungseinrichtung (7) im Gehäuse (2), über welche die Stromzuführungsleitung (6) aus der Gaszuführungsleitung (5) zur Bildung des Gasanschlussendstückes (9) und des Stromanschlussendstückes (8) geführt ist,
wobei der Stecker (4) am Gehäuse befestigt ist zur Verbindung mit der Steckbuchse in dem Gerät oder der zum Gerät führenden Anschlussleitung,
**gekennzeichnet dadurch, dass**
ein Filter (10) innerhalb des Gehäuses (2) im Gasanschlussendstück (9) angeordnet ist.

2. Anschlusseinrichtung nach Anspruch 1,
**dadurch gekennzeichnet, dass**
der Filter austauschbar ist.

3. Anschlusseinrichtung nach einem der vorangegangenen Ansprüche,
**dadurch gekennzeichnet, dass** die Abzweigungseinrichtung (7) eine erste Ausnehmung (11) zur steckartigen Aufnahme der Gaszuführungsleitung (5) mit der in dieser angeordneten Stromzuführungsleitung (6) aufweist.

4. Anschlusseinrichtung nach einem der vorangegangenen Ansprüche,
**dadurch gekennzeichnet,dass**
die Abzweigungseinrichtung (7) eine zweite Ausnehmung (12) zur steckartigen Aufnahme des Filters (10) aufweist.

5. Anschlusseinrichtung nach einem der vorangegangenen Ansprüche,
**dadurch gekennzeichnet,dass**
die Abzweigungseinrichtung (7) einen integrierten Kabelkanal (13) zur Aufnahme der Stromzuführungsleitung (6) aufweist.

6. Anschlusseinrichtung nach einem der vorangegangenen Ansprüche,
**dadurch gekennzeichnet,dass**
das elektrochirurgische Instrument zum einmaligen Gebrauch ausgebildet ist.

## Claims

1. APC probe that comprises at least one gas supply line (5), which can be connected to a socket in an appliance, or to a connecting line that leads to the appliance, by means of a plug connector (4), comprising
a housing (2) into which passes the gas supply line (5) with the power supply line (6) disposed therein and with a gas-connection end piece (9) and a power-connection end piece (8),
a branching device (7) in the housing (2), by way of which the power supply line (6) is guided out of the gas supply line (5) in order to form the gas-connection end piece (9) and the power-connection end piece (8),
wherein the plug connector (4) is fastened to the housing in order to make connection with the connector socket in the appliance or with the connecting line leading to the appliance,
**characterized in that**
a filter (10) is disposed within the housing (2) in the gas-connection end piece (9).

2. Connecting device according to Claim 1, **characterized in that** the filter is exchangeable.

3. Connecting device according to one of the preceding claims, **characterized in that** the branching device (7) comprises a first aperture (11) into which the gas supply line (5) with the power supply line (6) disposed therein is inserted, like a plug into a socket.

4. Connecting device according to one of the preceding claims, **characterized in that** the branching device (7) comprises a second aperture (12) into which the filter (10) is inserted, like a plug into a socket.

5. Connecting device according to one of the preceding claims, **characterized in that** the branching device (7) comprises an integrated cable channel (13) to accommodate the power supply line (6).

6. Connecting device according to one of the preceding claims, **characterized in that** the electrosurgical instrument is designed for single use.

## Revendications

1. Sonde APC, qui présente au moins une conduite d'arrivée de gaz (5), qui peut être reliée à une douille femelle dans un appareil ou une conduite de raccordement aboutissant à l'appareil au moyen d'une prise mâle (4), comprenant
un boîtier (2), dans lequel aboutit la conduite d'arrivée de gaz (5) avec la ligne d'arrivée de courant (6) disposée dans la conduite et avec une pièce d'extrémité de raccordement de gaz (9) et une pièce d'extrémité de raccordement d'électricité (8),
un dispositif de dérivation (7) dans le boîtier (2), au moyen duquel la ligne d'arrivée de courant (6) est guidée à la sortie de la conduite d'arrivée de gaz (5) pour former la pièce d'extrémité de raccordement de gaz (9) et la pièce d'extrémité de raccordement de courant (8),
la prise mâle (4) étant fixée sur le boîtier pour la liaison avec la douille femelle dans l'appareil ou la conduite de raccordement aboutissant à l'appareil,
**caractérisée en ce que**
un filtre (10) est disposé à l'intérieur du boîtier (2) dans la pièce d'extrémité de raccordement de gaz (9).

2. Dispositif de raccordement selon la revendication 1,
**caractérisé en ce que** le filtre est remplaçable

3. Dispositif de raccordement selon l'une quelconque des revendications précédentes,
**caractérisé en ce que** le dispositif de dérivation (7) présente un premier évidement (11) pour le logement en forme de prise de la conduite d'arrivée de gaz (5) avec la ligne d'arrivée de courant (6) disposée dans cette conduite.

4. Dispositif de raccordement selon l'une quelconque des revendications précédentes,
**caractérisé en ce que**
le dispositif de dérivation (7) présente un deuxième évidement (12) pour le logement en forme de prise du filtre (10).

5. Dispositif de raccordement selon l'une quelconque des revendications précédentes,
**caractérisé en ce que**
le dispositif de dérivation (7) présente un caniveau de câbles (13) intégré pour le logement de la ligne d'arrivée de courant (6).

6. Dispositif de raccordement selon l'une quelconque des revendications précédentes,
**caractérisé en ce que**
l'instrument pour l'électrochirurgie est conçu pour un usage unique.
